# EUROPEAN PATENT APPLICATION

(11) **EP 4 786 159 A1**
(43) Date of publication of application: **05.08.2026**
(21) Application number: 24870218.5
(22) Date of filing: 20.08.2024
(51) Int. Cl.: B32B 5/26, A61F 13/15, A61F 13/49, A61F 13/496, B32B 7/14, B32B 3/08, B32B 37/12

(54) **ELASTIC LAYER FOR WEARABLE SANITARY PRODUCT, AND PREPARATION METHOD THEREFOR**

(30) Priority: 27.09.2023 CN 202311264587
(71) Applicant: SHANDONG THRIVE MATERNITY AND INFANT PRODUCTS CO., LTD, Shandong 277500 (CN)
(72) Inventor: SUN, Feng, Zaozhuang, Shandong 277500 (CN); LIANG, Jinpan, Zaozhuang, Shandong 277500 (CN); WANG, Xishan, Zaozhuang, Shandong 277500 (CN)
(74) Representative: Cabinet Beau de Loménie
(86) International application number: PCT/CN2024/113226
(87) International publication number: WO 2025/066672

(57) **Abstract**

The present disclosure relates to an elastic layer for a wearable hygiene product and a preparation method therefor. The elastic layer for a wearable hygiene product comprises: N layers of base material which are stacked in sequence, wherein N is an integer greater than or equal to 3; and (N-1) groups of elastic bands, wherein each group of elastic bands among the (N-1) groups of elastic bands comprises a plurality of elastic bands; wherein one group of elastic bands is provided between two adjacent layers of base material, and a spacing between two adjacent elastic bands in each group of elastic bands ranges from 0.2 mm to 12 mm, and projections of two adjacent groups of elastic bands in a vertical direction are staggered, such that a combined spacing between adjacent elastic bands ranges from 0.1 mm to 6 mm. According to the elastic layer for a wearable hygiene product of the present disclosure, by arranging the projections of at least two groups of elastic bands being staggered in the vertical direction, the precision requirements for a spray-coating device are reduced while the manufactured elastic layer satisfies a smaller spacing, and thus reducing the cost and difficulty of spray-coating during the manufacturing process.

## Description

### Technical Field

The present disclosure relates to the field of hygiene care, and more particularly to an elastic layer for a wearable hygiene product and a preparation method therefor.

### Background Art

Wearable hygiene products have an elastic layer in the waistband to provide elasticity, allowing the diapers/pull-ups to fit users with different waist sizes.

The existing elastic layer generally comprises two layers of base material with multiple elastic bands (elastic strings) incorporated between them. To ensure elasticity, manufacturers typically minimize the spacing between adjacent elastic bands. However, during production, each individual strand must be coated with adhesive. The specific procedure involves placing multiple elastic bands into gaps of a dedicated spray-coating device-one strand per gap-followed by targeted spraying of adhesive onto the elastic bands within the gaps. This method presents the following drawbacks:
The smaller the spacing between elastic bands is, the higher the precision requirements for the gaps of the spray-coating device are. It is difficult for general spray-coating devices to achieve this precision requirement, which increases the cost and difficulty of spray-coating.

The information disclosed in the background section of the present invention is only intended to enhance an understanding of the general background of the present invention, and should not be construed as an admission or suggestion in any form that such information constitutes prior art known to those skilled in the art.

### Summary

The present disclosure aims to provide an elastic layer for a wearable hygiene product, and a preparation method therefor, and by arranging the projections of a plurality of first elastic bands (elastic strings) and a plurality of second elastic bands (elastic strings) being staggered in a vertical direction (in an up-down direction), the precision requirements for a spray-coating device are reduced while the manufactured elastic layer satisfies a smaller spacing, and thus reducing the cost and difficulty of spray-coating during the manufacturing process.

According to a first aspect of the present disclosure, there is provided an elastic layer for a wearable hygiene product, characterized in comprising: N layers of base material which are stacked in sequence, wherein N is an integer greater than or equal to 3; and (N-1) groups of elastic bands, wherein each group of elastic bands among the (N-1) groups of elastic bands comprises a plurality of elastic bands; wherein one group of elastic bands is provided between two adjacent layers of base material, and a spacing between two adjacent elastic bands in each group of elastic bands ranges from 0.2 mm to 12 mm, and projections of two adjacent groups of elastic bands in a vertical direction are staggered, such that a combined spacing between adjacent elastic bands ranges from 0.1 mm to 6 mm.

Preferably, the elastic layer for a wearable hygiene product comprises: a first base material, a second base material, and a third base material which are stacked in sequence; a plurality of first elastic bands forming a first group of elastic bands, which are arranged between the first base material and the second base material, wherein a spacing between two adjacent elastic bands in the plurality of first elastic bands ranges from 0.2 mm to 12 mm; and a plurality of second elastic bands forming a second group of elastic bands, which are arranged between the second base material and the third base material, wherein a spacing between two adjacent elastic bands in the plurality of second elastic bands ranges from 0.2 mm to 12 mm; wherein projections of the plurality of first elastic bands and the plurality of second elastic bands in a vertical direction are staggered, such that a combined spacing between adjacent first and second elastic bands ranges from 0.1 mm to 6 mm.

Preferably, the elastic layer for a wearable hygiene product comprises: a first base material, a second base material, a third base material, and a fourth base material which are stacked in sequence; a plurality of first elastic bands forming a first group of elastic bands, which are arranged between the first base material and the second base material, wherein a spacing between two adjacent elastic bands in the plurality of first elastic bands ranges from 0.2 mm to 12 mm; a plurality of second elastic bands forming a second group of elastic bands, which are arranged between the second base material and the third base material, wherein a spacing between two adjacent elastic bands in the plurality of second elastic bands ranges from 0.2 mm to 12 mm; and a plurality of third elastic bands forming a third group of elastic bands, which are arranged between the third base material and the fourth base material, wherein a spacing between two adjacent elastic bands in the plurality of third elastic bands ranges from 0.2 mm to 12 mm; wherein projections of the plurality of first elastic bands, the plurality of second elastic bands, and the plurality of third elastic bands in a vertical direction are staggered, such that a combined spacing between adjacent elastic bands ranges from 0.1 mm to 6 mm.

Preferably, the elastic layer for a wearable hygiene product comprises: a first base material, a second base material, a third base material, a fourth base material, and a fifth base material which are stacked in sequence; a plurality of first elastic bands forming a first group of elastic bands, which are arranged between the first base material and the second base material, wherein a spacing between two adjacent elastic bands in the plurality of first elastic bands ranges from 0.2 mm to 12 mm; a plurality of second elastic bands forming a second group of elastic bands, which are arranged between the second base material and the third base material, wherein a spacing between two adjacent elastic bands in the plurality of second elastic bands ranges from 0.2 mm to 12 mm; a plurality of third elastic bands forming a third group of elastic bands, which are arranged between the third base material and the fourth base material, wherein a spacing between two adjacent elastic bands in the plurality of third elastic bands ranges from 0.2 mm to 12 mm; and a plurality of fourth elastic bands forming a fourth group of elastic bands, which are arranged between the fourth base material and the fifth base material, wherein a spacing between two adjacent elastic bands in the plurality of fourth elastic bands ranges from 0.2 mm to 12 mm; wherein projections of the plurality of first elastic bands, the plurality of second elastic bands, the plurality of third elastic bands, and the plurality of fourth elastic bands in a vertical direction are staggered, such that a combined spacing between adjacent elastic bands ranges from 0.1 mm to 6 mm.

Preferably, the plurality of first elastic bands are distributed at equal intervals, and the plurality of second elastic bands are distributed at equal intervals.

Preferably, the plurality of first elastic bands are distributed at equal intervals, the plurality of second elastic bands are distributed at equal intervals, and the plurality of third elastic bands are distributed at equal intervals.

Preferably, the plurality of first elastic bands are distributed at equal intervals, the plurality of second elastic bands are distributed at equal intervals, the plurality of third elastic bands are distributed at equal intervals, and the plurality of fourth elastic bands are distributed at equal intervals.

Preferably, the elastic band has an average linear density of 10 dtex to 440 dtex.

Preferably, at least one layer of (N-2) layers of base material in the N layers of base material is a discontinuous base material.

According to a second aspect of the present disclosure, there is provided a method for preparing an elastic layer for a wearable hygiene product, comprising: preparing N layers of base material, wherein N is an integer greater than or equal to 3; forming a group of elastic bands between any two adjacent layers of base material, each group of elastic bands comprising a plurality of elastic bands, wherein a spacing between two adjacent elastic bands in each group of elastic bands ranges from 0.2 mm to 12 mm; and stacking the N layers of base material in sequence, wherein projections of (N-1) groups of elastic bands in a vertical direction are staggered, such that a combined spacing between adjacent elastic bands ranges from 0.1 mm to 6 mm.

Preferably, prior to forming a group of elastic bands between any two adjacent layers of base material, the method further comprises: spray-coating adhesive on the plurality of elastic bands; wherein the elastic bands are able to be bonded to its two adjacent layers of base material through adhesive, and each layer of base material is bonded to an adjacent layer of base material through the adhesive spray-coated on the elastic bands.

Preferably, prior to forming a group of elastic bands between any two adjacent layers of base material, the method further comprises: spray-coating adhesive on one of surfaces of two adjacent layers of base material that will contact with each other; wherein the elastic bands are able to be bonded to its two adjacent layers of base material through the adhesive, and the two adjacent layers of base material are bonded through the adhesive.

Preferably, the method for preparing an elastic layer for a wearable hygiene product comprises: preparing three layers of base material which comprise a first base material, a second base material, and a third base material; laying a plurality of first elastic bands on an upper surface of the first base material, wherein a spacing between two adjacent elastic bands in the plurality of first elastic bands ranges from 0.2 mm to 12 mm; laying a plurality of second elastic bands on a lower surface of the third base material, wherein a spacing between two adjacent elastic bands in the plurality of second elastic bands ranges from 0.2 mm to 12 mm; stacking the second base material on a side of the first base material where the first elastic bands are laid; and stacking a side of the third base material where the second elastic bands are laid above the second base material, such that projections of the plurality of first elastic bands and the plurality of second elastic bands in a vertical direction are staggered, and a combined spacing between adjacent first and second elastic bands ranges from 0.1 mm to 6 mm.

Preferably, the method for preparing an elastic layer for a wearable hygiene product comprises: preparing three layers of base material which comprise a first base material, a second base material, and a third base material; laying a plurality of first elastic bands on a lower surface of the second base material, and laying a plurality of second elastic bands on an upper surface of the second base material, wherein a spacing between two adjacent elastic bands in the plurality of first elastic bands ranges from 0.2 mm to 12 mm, and a spacing between two adjacent elastic bands in the plurality of second elastic bands ranges from 0.2 mm to 12 mm, and projections of the plurality of first elastic bands and the plurality of second elastic bands in a vertical direction are staggered, and a combined spacing between adjacent first and second elastic bands ranges from 0.1 mm to 6 mm; stacking the second base material on the first base material; and stacking the third base material on the second base material.

Preferably, the method for preparing an elastic layer for a wearable hygiene product comprises: preparing three layers of base material which comprise a first base material, a second base material, and a third base material; laying a plurality of first elastic bands on an upper surface of the first base material, wherein a spacing between two adjacent elastic bands in the plurality of first elastic bands ranges from 0.2 mm to 12 mm; stacking the second base material on the first base material; laying a plurality of second elastic bands on an upper surface of the second base material, wherein a spacing between two adjacent elastic bands in the plurality of second elastic bands ranges from 0.2 mm to 12 mm, such that projections of the plurality of first elastic bands and the plurality of second elastic bands in a vertical direction are staggered, and a combined spacing between adjacent first and second elastic bands ranges from 0.1 mm to 6 mm; and stacking the third base material on the second base material.

Preferably, the method for preparing an elastic layer for a wearable hygiene product comprises: preparing four layers of base material which comprise a first base material, a second base material, a third base material, and a fourth base material; laying a plurality of first elastic bands on an upper surface of the first base material, wherein a spacing between two adjacent elastic bands in the plurality of first elastic bands ranges from 0.2 mm to 12 mm; laying a plurality of second elastic bands on a lower surface of the third base material, and laying a plurality of third elastic bands on an upper surface of the third base material, wherein a spacing between two adjacent elastic bands in the plurality of second elastic bands ranges from 0.2 mm to 12 mm, and a spacing between two adjacent elastic bands in the plurality of third elastic bands ranges from 0.2 mm to 12 mm; stacking the second base material on the first base material; stacking a side of the third base material where the second elastic bands are laid on the second base material; and stacking the fourth base material on the third base material, such that projections of the plurality of first elastic bands, the plurality of second elastic bands, and the plurality of third elastic bands in a vertical direction are staggered, and a combined spacing between adjacent first, second, and third elastic bands ranges from 0.1 mm to 6 mm.

Preferably, the method for preparing an elastic layer for a wearable hygiene product comprises: preparing four layers of base material which comprise a first base material, a second base material, a third base material, and a fourth base material; laying a plurality of first elastic bands on a lower surface of the second base material, laying a plurality of second elastic bands on an upper surface of the second base material, laying a plurality of third elastic bands on a lower surface of the third base material, and laying a plurality of fourth elastic bands on an upper surface of the fourth base material, wherein a spacing between two adjacent elastic bands in the plurality of first elastic bands ranges from 0.2 mm to 12 mm, a spacing between two adjacent elastic bands in the plurality of second elastic bands ranges from 0.2 mm to 12 mm, and a spacing between two adjacent elastic bands in the plurality of third elastic bands ranges from 0.2 mm to 12 mm, wherein projections of the plurality of first elastic bands, the plurality of second elastic bands, and the plurality of third elastic bands in a vertical direction are staggered, and a combined spacing between adjacent first, second, and third elastic bands ranges from 0.1 mm to 6 mm; stacking the second base material on the first base material; stacking the third base material on the second base material; and stacking the fourth base material on the third base material.

Preferably, the method for preparing an elastic layer for a wearable hygiene product comprises: preparing four layers of base material which comprise a first base material, a second base material, a third base material, and a fourth base material; laying a plurality of first elastic bands on an upper surface of the first base material, wherein a spacing between two adjacent elastic bands in the plurality of first elastic bands ranges from 0.2 mm to 12 mm; stacking the second base material on the first base material; laying a plurality of second elastic bands on an upper surface of the second base material, wherein a spacing between two adjacent elastic bands in the plurality of second elastic bands ranges from 0.2 mm to 12 mm; stacking the third base material on the second base material; laying a plurality of third elastic bands on an upper surface of the third base material, wherein a spacing between two adjacent elastic bands in the plurality of third elastic bands ranges from 0.2 mm to 12 mm, wherein projections of the plurality of first elastic bands, the plurality of second elastic bands, and the plurality of third elastic bands in a vertical direction are staggered, and a combined spacing between adjacent first, second and third elastic bands ranges from 0.1 mm to 6 mm; and stacking the fourth base material on the third base material.

Preferably, the method for preparing an elastic layer for a wearable hygiene product comprises: preparing five layers of base material which comprise a first base material, a second base material, a third base material, a fourth base material, and a fifth base material; laying a plurality of first elastic bands on an upper surface of the first base material, wherein a spacing between two adjacent elastic bands in the plurality of first elastic bands ranges from 0.2 mm to 12 mm; stacking the second base material on the first base material; laying a plurality of second elastic bands on an upper surface of the second base material, wherein a spacing between two adjacent elastic bands in the plurality of second elastic bands ranges from 0.2 mm to 12 mm; stacking the third base material on the second base material; laying a plurality of third elastic bands on an upper surface of the third base material, wherein a spacing between two adjacent elastic bands in the plurality of third elastic bands ranges from 0.2 mm to 12 mm; stacking the fourth base material on the third base material; laying a plurality of fourth elastic bands on an upper surface of the fourth base material, wherein a spacing between two adjacent elastic bands in the plurality of fourth elastic bands ranges from 0.2 mm to 12 mm, wherein projections of the plurality of first elastic bands, the plurality of second elastic bands, the plurality of third elastic bands, and the plurality of fourth elastic bands in a vertical direction are staggered, and a combined spacing between adjacent first, second, third, and fourth elastic bands ranges from 0.1 mm to 6 mm; and stacking the fifth base material on the fourth base material.

According to the elastic layer for a wearable hygiene product of the present disclosure, by arranging the projections of at least two groups of elastic bands being staggered in the vertical direction (in an up-down direction), the precision requirements for a spray-coating device are reduced while the manufactured elastic layer satisfies a smaller spacing, and thus reducing the cost and difficulty of spray-coating during the manufacturing process.

The methods and devices of the present disclosure have other characteristics and advantages that will be apparent from the accompanying drawings and subsequent specific embodiments incorporated herein, or will be described in detail in the accompanying drawings and subsequent embodiments, which together serve to explain the particular principles of the present disclosure.

### Brief Description of Drawings

Figure 1A is a schematic diagram of the internal structure of a first elastic layer for a wearable hygiene product provided by an embodiment of the present disclosure;
Figure 1B is an exploded perspective view corresponding to Figure 1A;
Figure 2A is a schematic diagram of the internal structure of a second elastic layer for a wearable hygiene product provided by an embodiment of the present disclosure;
Figure 2B is an exploded perspective view corresponding to Figure 2A;
Figure 3A is a schematic diagram of the internal structure of a third elastic layer for a wearable hygiene product provided by an embodiment of the present disclosure;
Figure 3B is an exploded perspective view corresponding to Figure 3A;
Figure 4 is a schematic diagram of the internal structure of a fourth elastic layer for a wearable hygiene product provided by an embodiment of the present disclosure;
Figure 5A is a schematic diagram of the internal structure of a fifth elastic layer for a wearable hygiene product provided by an embodiment of the present disclosure;
Figure 5B is a schematic diagram of the internal structure of a sixth elastic layer for a wearable hygiene product provided by an embodiment of the present disclosure;
Figure 5C is a schematic diagram of the internal structure of a seventh elastic layer for a wearable hygiene product provided by an embodiment of the present disclosure;
Figure 5D is a schematic diagram of the internal structure of an eighth elastic layer for a wearable hygiene product provided by an embodiment of the present disclosure;
Figure 5E is a schematic diagram of the internal structure of a ninth elastic layer for a wearable hygiene product provided by an embodiment of the present disclosure;
Figure 5F is a schematic diagram of the internal structure of a tenth elastic layer for a wearable hygiene product provided by an embodiment of the present disclosure;
Figure 5G is a schematic diagram of the internal structure of an eleventh elastic layer for a wearable hygiene product provided by an embodiment of the present disclosure;
Figure 5H is a schematic diagram of the internal structure of a twelfth elastic layer for a wearable hygiene product provided by an embodiment of the present disclosure;
Figure 5I is a schematic diagram of the internal structure of a thirteenth elastic layer for a wearable hygiene product provided by an embodiment of the present disclosure;
Figure 6 is a schematic diagram of the internal structure of a fourteenth elastic layer for a wearable hygiene product provided by an embodiment of the present disclosure;
Figure 7A is a schematic diagram I of discontinuous base material;
Figure 7B is a schematic diagram II of discontinuous base material;
Figure 8 is a schematic flowchart of a first method for preparing an elastic layer for a wearable hygiene product provided by an embodiment of the present disclosure; and
Figure 9 is a schematic flowchart of a second method for preparing an elastic layer for a wearable hygiene product provided by an embodiment of the present disclosure.

**List of reference numerals:**

| | | | |
|---|---|---|---|
| 101: | first base material | 102: | second base material |
| 103: | third base material | 104: | fourth base material |
| 105: | fifth base material | | |
| 201: | first elastic band | 202: | second elastic band |
| 203: | third elastic band | 204: | fourth elastic band |

It should be understood that the accompanying drawings are not necessarily drawn to scale, but rather present simplified representations of various features to illustrate the basic principles of the present disclosure. The specific design features disclosed in the present disclosure, including specific dimensions, directions, positions, and shapes, will be partially determined by the specific application and usage environment.

In these drawings, the same reference numerals refer to the same or equivalent parts of the present disclosure throughout the drawings.

### Detailed Description of Embodiments

Reference will now be made in detail to various embodiments of the present disclosure, examples of which are presented in the accompanying drawings and described as follows. Although the present disclosure is described in conjunction with exemplary embodiments, it should be understood that this description is not intended to limit the present disclosure to these exemplary embodiments. On the contrary, the present disclosure aims to cover not only these exemplary embodiments, but also various substitutions, modifications, equivalents, and other embodiments that can be included within the spirit and scope of the present disclosure as defined by the appended claims..

When a component is referred to as being "above" or "on" another component, the component may be in contact with the other component or there may be intermediate components.

The wearable hygiene products mentioned in the embodiments of the present disclosure include, but are not limited to, baby diapers/pull-ups, women's overnight pants, and adult diapers/pull-ups.

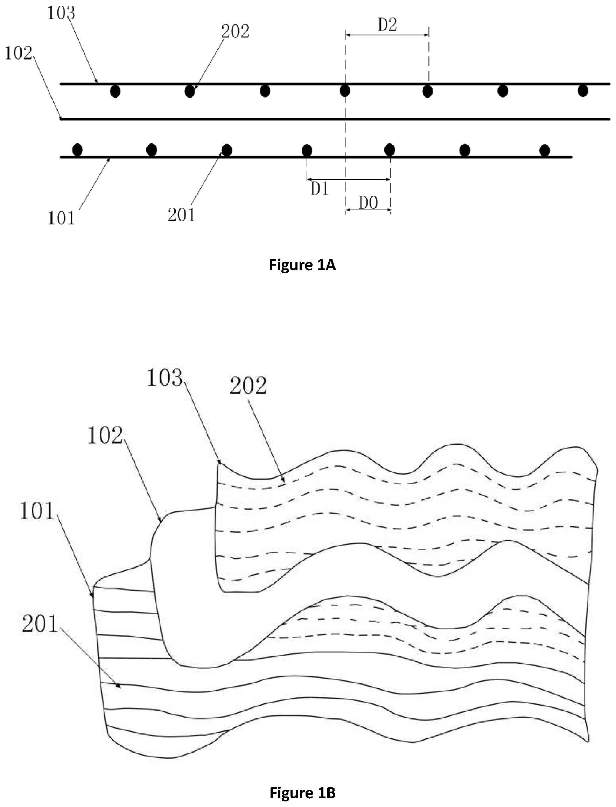

The embodiments of Figures 1A to 4 all include three layers of base material and two groups of elastic bands.

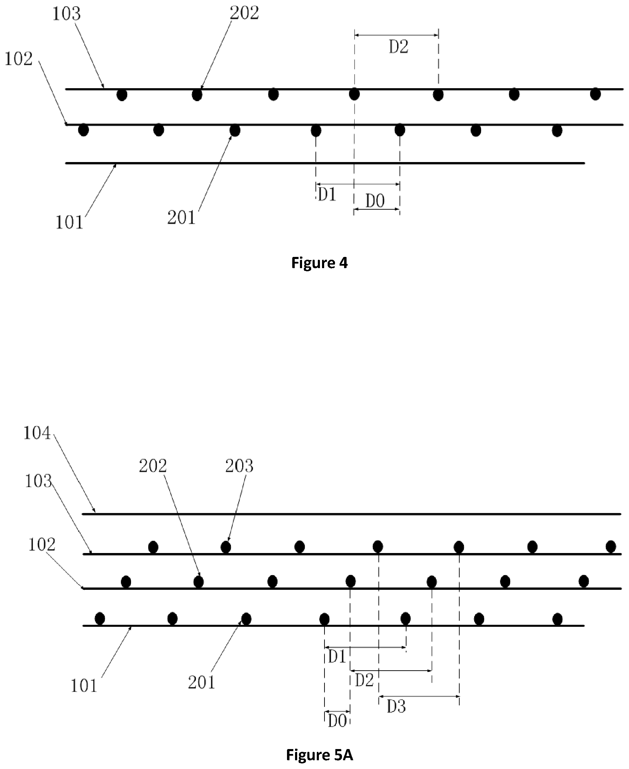

The embodiments of Figures 5A to 5I all include four layers of base material and three groups of elastic bands.

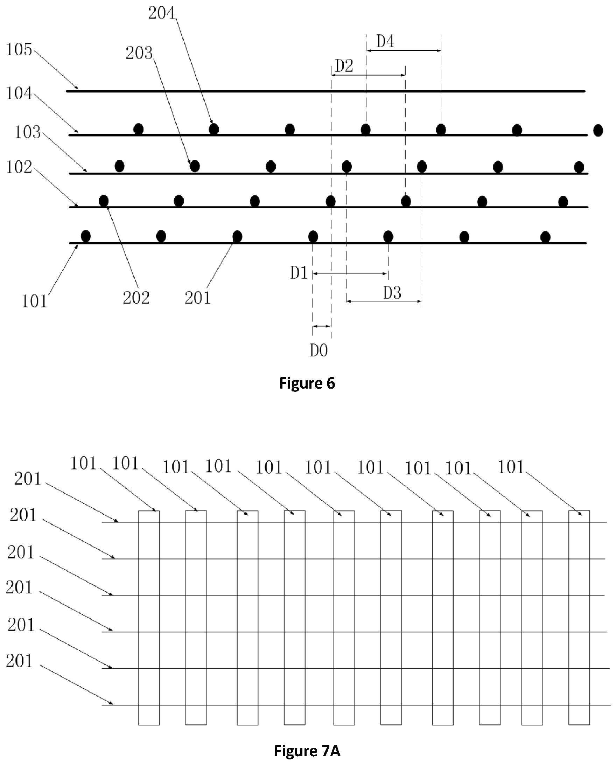

The embodiment of Figure 6 includes five layers of base material and four groups of elastic bands.

The embodiments shown in Figures 1A, 2A, 3A, 4, 5A to 5I, and 6 are all cross-sectional views.

The elastic layer for a wearable hygiene product according to embodiments of the present disclosure will be described below with reference to Figures 1A to 6.

The elastic layer for a wearable hygiene product in the embodiments of the present disclosure includes N layers of base material and (N-1) groups of elastic bands.

The N layers of base material are stacked in sequence, wherein N is an integer greater than or equal to 3. Each group of elastic bands among the (N-1) groups of elastic bands comprises a plurality of elastic bands.

One group of elastic bands is provided between two adjacent layers of base material, and the spacing between two adjacent elastic bands in each group of elastic bands ranges from 0.2 mm to 12 mm, and the projections of two adjacent groups of elastic bands in a vertical direction are staggered, such that a combined spacing between adjacent elastic bands ranges from 0.1 mm to 6 mm.

According to the elastic layer for a wearable hygiene product of the embodiments of the present disclosure, by arranging the projections of at least two groups of elastic bands being staggered in the vertical direction, the precision requirements for a spray-coating device are reduced while the manufactured elastic layer satisfies a smaller spacing, thus reducing the cost and difficulty of spray-coating during the manufacturing process.

### Example 1

Figure 1A is a schematic diagram of the internal structure of a first elastic layer for a wearable hygiene product provided by an embodiment of the present disclosure; Figure 1B is an exploded perspective view corresponding to Figure 1A.

As shown in Figures 1A and 1B, the elastic layer for a wearable hygiene product in Example 1 comprises: a first base material 101, a second base material 102, a third base material 103, a plurality of first elastic bands 201, and a plurality of second elastic bands 202.

The first base material 101, the second base material 102, and the third base material 103 are stacked in sequence.

The plurality of first elastic bands 201 form a first group of elastic bands, which are arranged between the first base material 101 and the second base material 102, and the spacing D1 between two adjacent elastic bands in the plurality of first elastic bands 201 ranges from 0.2 mm to 12 mm (e.g., 0.2 mm, 0.3 mm, 0.4 mm, 0.5 mm, 0.6 mm, 0.7 mm, 0.8 mm, 0.9 mm, 1 mm, 1.1 mm, 1.2 mm, 1.3 mm, 1.4 mm, 1.5 mm, 1.6 mm, 1.7 mm, 1.8 mm, 1.9 mm, 2 mm, 3 mm, 4 mm, 5 mm, 6 mm, 7 mm, 8 mm, 9 mm, 10 mm, 11 mm, 12 mm). Preferably, the spacing D1 between two adjacent elastic bands in the plurality of first elastic bands 201 ranges from 2 mm to 12 mm.

The plurality of second elastic bands 202 form a second group of elastic bands, which are arranged between the second base material 102 and the third base material 103, and the spacing D2 between two adjacent elastic bands in the plurality of second elastic bands 202 ranges from 0.2 mm to 12 mm (e.g., 0.2 mm, 0.3 mm, 0.4 mm, 0.5 mm, 0.6 mm, 0.7 mm, 0.8 mm, 0.9 mm, 1 mm, 1.1 mm, 1.2 mm, 1.3 mm, 1.4 mm, 1.5 mm, 1.6 mm, 1.7 mm, 1.8 mm, 1.9 mm, 2 mm, 3 mm, 4 mm, 5 mm, 6 mm, 7 mm, 8 mm, 9 mm, 10 mm, 11 mm, 12 mm). Preferably, the spacing D2 between two adjacent elastic bands in the plurality of second elastic bands 202 ranges from 2 mm to 12 mm.

The projections of the plurality of first elastic bands 201 and the plurality of second elastic bands 202 in a vertical direction are staggered, such that the combined spacing D0 between adjacent first elastic bands 201 and second elastic bands 202 ranges from 0.1 mm to 6 mm (e.g., 0.1 mm, 0.2 mm, 0.3 mm, 0.4 mm, 0.5 mm, 0.6 mm, 0.7 mm, 0.8 mm, 0.9 mm, 1 mm, 1.5 mm, 2 mm, 2.5 mm, 3 mm, 3.5 mm, 4 mm, 4.5 mm, 5 mm, 5.5 mm, 6 mm). Preferably, the combined spacing D0 between adjacent first elastic bands 201 and second elastic bands 202 ranges from 1 mm to 6mm.

In Example 1, by arranging the projections of the plurality of first elastic bands 201 and the plurality of second elastic bands 202 being staggered in a vertical direction, the precision requirements for a spray-coating device are reduced while the manufactured elastic layer satisfies a smaller spacing, thus reducing the cost and difficulty of spray-coating during the manufacturing process.

For example, in the prior art, when the spacing between adjacent elastic bands is 3 mm, the gap precision of the spray-coating device is also required to be 3 mm.

When the embodiment of Example 1 is adopted, when the spacing between adjacent first elastic bands 201 and second elastic bands 202 is also 3 mm (i.e. D0 is 3 mm), due to the staggered projection arrangements of the plurality of first elastic bands 201 and the plurality of second elastic bands 202 in the vertical direction, it is only necessary for the spacing between two adjacent elastic bands in the plurality of first elastic bands 201 to be 6 mm (i.e., D1 is 6 mm), and it is only necessary for the spacing between two adjacent elastic bands in the plurality of second elastic bands 202 to be 6 mm (i.e., D2 is 6 mm). At this time, the gap precision of the spray-coating device is only required to be 6 mm.

In exemplary embodiments, the first base material 101/second base material 102/third base material 103 can be selected from non-woven fabrics such as spunlace fabric, spunbond fabric, or spunmelt fabric.

When a diaper is worn, the first base material 101, the second base material 102, the third base material 103, the plurality of first elastic bands 201, and the plurality of second elastic bands 202 of the elastic layer for a wearable hygiene product extend in the circumferential direction of the waist of the human body.

It should be noted that Figures 1A to 6 in the present disclosure only schematically illustrate the approximate shapes of each layer and the approximate positional relationships between each layer. To better distinguish the boundaries between layers, gaps are schematically illustrated between the layers in Figures 1A to 6, whereas in actual products, the first base material 101, the second base material 102, and the third base material 103 are bonded together without gaps.

In exemplary embodiments, the peel strength between the first base material 101 and the second base material 102 is about 1 N/cm to about 15 N/cm (e.g., 1 N/cm, 2 N/cm, 3 N/cm, 4 N/cm, 5 N/cm, 6 N/cm, 7 N/cm, 8 N/cm, 9 N/cm, 10 N/cm, 11 N/cm, 12 N/cm, 13 N/cm, 14 N/cm, 15 N/cm).

In exemplary embodiments, the peel strength between the second base material 102 and the third base material 103 is about 1 N/cm to about 15 N/cm (e.g., 1 N/cm, 2 N/cm, 3 N/cm, 4 N/cm, 5 N/cm, 6 N/cm, 7 N/cm, 8 N/cm, 9 N/cm, 10 N/cm, 11 N/cm, 12 N/cm, 13 N/cm, 14 N/cm, 15 N/cm).

In exemplary embodiments, adhesive is spray-coated onto the first elastic bands 201. The first base material 101 is bonded to the second base material 102 through the adhesive spray-coated on the first elastic bands 201.

In exemplary embodiments, adhesive is spray-coated onto the second elastic bands 202. The second base material 102 is bonded to the third base material 103 through the adhesive spray-coated on the second elastic bands 202.

In addition to adhesive bonding, the first elastic bands 201, the first base material 101, and the second base material 102, as well as the second elastic bands 202, the second base material 102, and the third base material 103, can also be bonded together by ultrasonic welding.

In an embodiment, as shown in Figure 1A, the plurality of first elastic bands 201 are distributed at equal intervals. In another embodiment, the plurality of first elastic bands 201 can also be distributed at unequal intervals (not illustrated in the figures).

In an embodiment, as shown in Figure 1A, the plurality of second elastic bands 202 are distributed at equal intervals. In another embodiment, the plurality of second elastic bands 202 can also be distributed at unequal intervals (not illustrated in the figures).

In exemplary embodiments, the first elastic band 201 has an average linear density of 10 dtex to 940 dtex (e.g., 10 dtex, 50 dtex, 100 dtex, 150 dtex, 200 dtex, 250 dtex, 300 dtex, 350 dtex, 400 dtex, 440 dtex, 500 dtex, 600 dtex, 700 dtex, 800 dtex, 940 dtex), and the second elastic band 202 has an average linear density of 10 dtex to 940 dtex (e.g., 10 dtex, 50 dtex, 100 dtex, 150 dtex, 200 dtex, 250 dtex, 300 dtex, 350 dtex, 400 dtex, 440 dtex, 500 dtex, 600 dtex, 700 dtex, 800 dtex, 940 dtex).

In exemplary embodiments, the first elastic band 201 has a tensile range of 100% to 400% (e.g., 100%, 150%, 200%, 250%, 300%, 350%, 400%).

In exemplary embodiments, the second elastic band 202 has a tensile range of 100% to 400% (e.g., 100%, 150%, 200%, 250%, 300%, 350%, 400%).

In exemplary embodiments, the elastic layer includes 10 to 130 (e.g. 10, 20, 30, 40, 50, 60, 70, 80, 90, 100, 110, 120, 130) first elastic bands 201.

In exemplary embodiments, the elastic layer includes 10 to 130 (e.g. 10, 20, 30, 40, 50, 60, 70, 80, 90, 100, 110, 120, 130) second elastic bands 202.

In exemplary embodiments, the first base material 101 and the second base material 102 are bonded together through a first bonding portion, and the second base material 102 and the third base material 103 are bonded together through a second bonding portion.

### Example 2

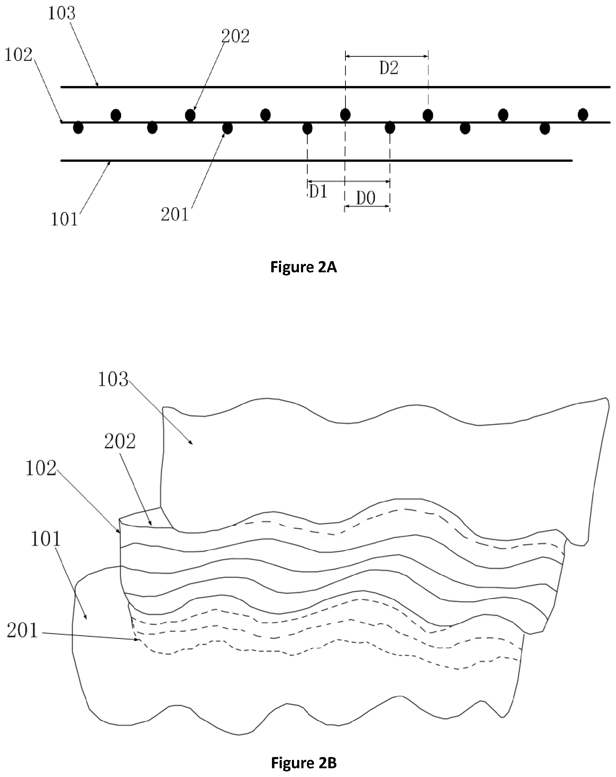

Figure 2A is a schematic diagram of the internal structure of a first elastic layer for a wearable hygiene product provided by an embodiment of the present disclosure; Figure 2B is an exploded perspective view corresponding to Figure 2A.

As shown in Figures 1A and 1B, the elastic layer for a wearable hygiene product in Example 2 comprises: a first base material 101, a second base material 102, a third base material 103, a plurality of first elastic bands 201, and a plurality of second elastic bands 202.

### Example 3

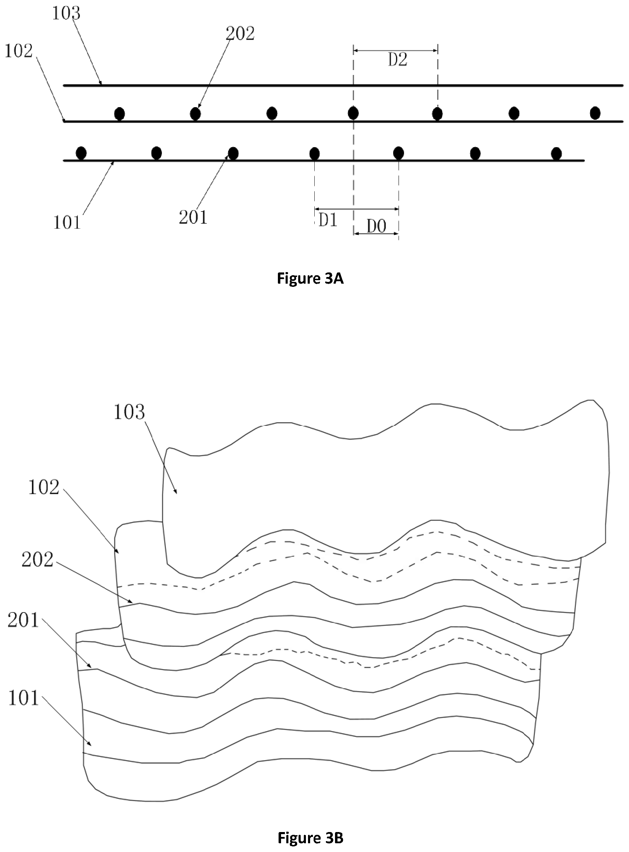

Figure 3A is a schematic diagram of the internal structure of a first elastic layer for a wearable hygiene product provided by an embodiment of the present disclosure; Figure 3B is an exploded perspective view corresponding to Figure 3A.

As shown in Figures 3A and 3B, the elastic layer for a wearable hygiene product in Example 3 comprises: a first base material 101, a second base material 102, a third base material 103, a plurality of first elastic bands 201, and a plurality of second elastic bands 202.

### Example 4

Figure 4 is a schematic diagram of the internal structure of a first elastic layer for a wearable hygiene product provided by an embodiment of the present disclosure;
As shown in Figure 4, the elastic layer for a wearable hygiene product in Example 4 comprises: a first base material 101, a second base material 102, a third base material 103, a plurality of first elastic bands 201, and a plurality of second elastic bands 202.

The layered structures of the finished elastic layers in Examples 1, 2, 3, and 4 are the same, with the only difference being the order of the preparation methods, which will be explained in detail in the following preparation methods.

### Example 5

As shown in Figure 5A, the elastic layer for a wearable hygiene product in Example 5 comprises: a first base material 101, a second base material 102, a third base material 103, a fourth base material 104, a plurality of first elastic bands 201, a plurality of second elastic bands 202, and a plurality of third elastic bands 203.

The first base material 101, the second base material 102, the third base material 103, and the fourth base material 104 are stacked in sequence.

The plurality of first elastic bands 201 form a first group of elastic bands, which are arranged between the first base material 101 and the second base material 102, and the spacing between two adjacent elastic bands in the plurality of first elastic bands 201 ranges from 0.2 mm to 12 mm.

The plurality of second elastic bands 202 form a second group of elastic bands, which are arranged between the second base material 102 and the third base material 103, and the spacing between two adjacent elastic bands in the plurality of second elastic bands 202 ranges from 0.2 mm to 12 mm.

The plurality of third elastic bands 203 form a third group of elastic bands, which are arranged between the third base material 103 and the fourth base material 104, and the spacing between two adjacent elastic bands in the plurality of third elastic bands 203 ranges from 0.2 mm to 12 mm.

The projections of the plurality of first elastic bands 201, the plurality of second elastic bands 202, and the plurality of third elastic bands 203 in a vertical direction are staggered, such that the combined spacing D0 between adjacent elastic bands ranges from 0.1 mm to 6 mm.

### Example 6

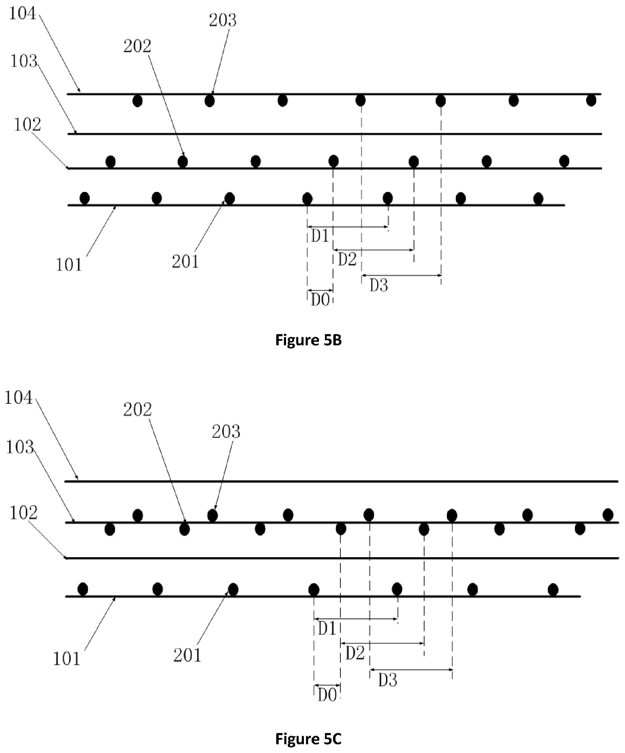

As shown in Figure 5B, the elastic layer for a wearable hygiene product in Example 6 comprises: a first base material 101, a second base material 102, a third base material 103, a fourth base material 104, a plurality of first elastic bands 201, a plurality of second elastic bands 202, and a plurality of third elastic bands 203.

### Example 7

As shown in Figure 5C, the elastic layer for a wearable hygiene product in Example 7 comprises: a first base material 101, a second base material 102, a third base material 103, a fourth base material 104, a plurality of first elastic bands 201, a plurality of second elastic bands 202, and a plurality of third elastic bands 203.

### Example 8

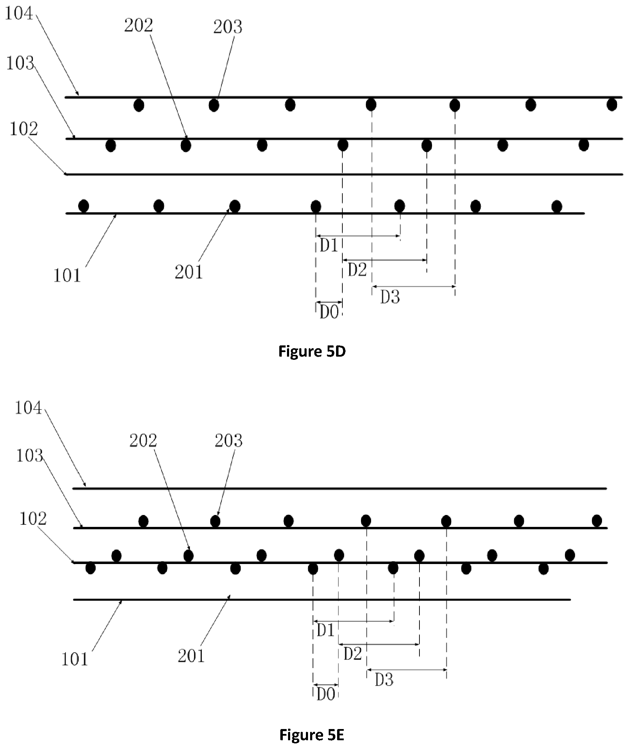

As shown in Figure 5D, the elastic layer for a wearable hygiene product in Example 8 comprises: a first base material 101, a second base material 102, a third base material 103, a fourth base material 104, a plurality of first elastic bands 201, a plurality of second elastic bands 202, and a plurality of third elastic bands 203.

### Example 9

As shown in Figure 5E, the elastic layer for a wearable hygiene product in Example 9 comprises: a first base material 101, a second base material 102, a third base material 103, a fourth base material 104, a plurality of first elastic bands 201, a plurality of second elastic bands 202, and a plurality of third elastic bands 203.

### Example 10

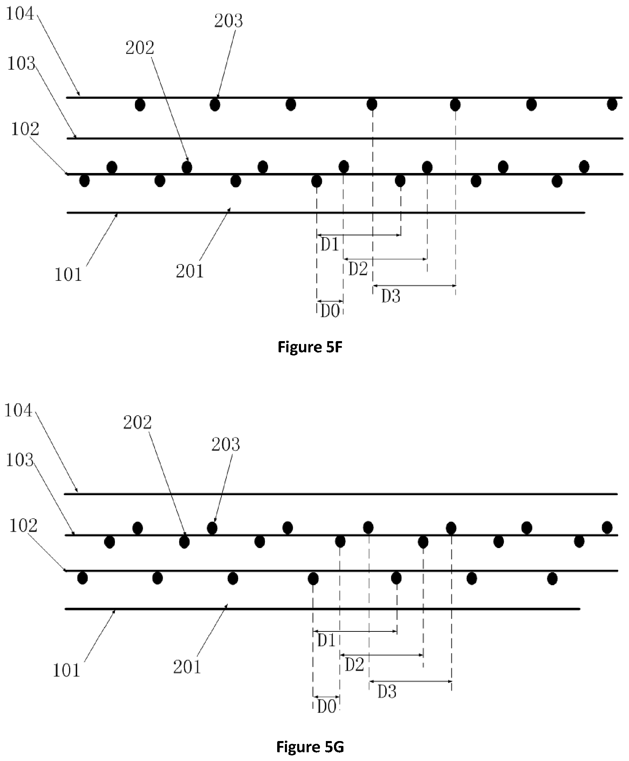

As shown in Figure 5F, the elastic layer for a wearable hygiene product in Example 10 comprises: a first base material 101, a second base material 102, a third base material 103, a fourth base material 104, a plurality of first elastic bands 201, a plurality of second elastic bands 202, and a plurality of third elastic bands 203.

### Example 11

As shown in Figure 5G, the elastic layer for a wearable hygiene product in Example 11 comprises: a first base material 101, a second base material 102, a third base material 103, a fourth base material 104, a plurality of first elastic bands 201, a plurality of second elastic bands 202, and a plurality of third elastic bands 203.

### Example 12

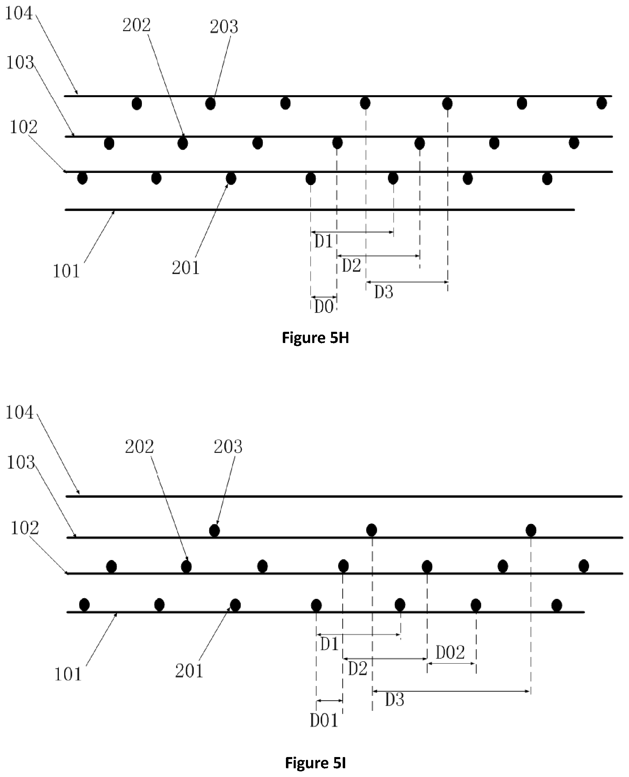

As shown in Figure 5H, the elastic layer for a wearable hygiene product in Example 12 comprises: a first base material 101, a second base material 102, a third base material 103, a fourth base material 104, a plurality of first elastic bands 201, a plurality of second elastic bands 202, and a plurality of third elastic bands 203.

The layered structures of the finished elastic layers in Examples 5 to 12 are the same, with the only difference being the order of preparation methods, which will be explained in detail in the following preparation methods.

### Example 13

As shown in Figure 5I, the elastic layer for a wearable hygiene product in Example 13 comprises: a first base material 101, a second base material 102, a third base material 103, a fourth base material 104, a plurality of first elastic bands 201, a plurality of second elastic bands 202, and a plurality of third elastic bands 203.

The first base material 101, the second base material 102, the third base material 103, and the fourth base material 104 are stacked in sequence.

The plurality of first elastic bands 201 form a first group of elastic bands, which are arranged between the first base material 101 and the second base material 102, and the spacing between two adjacent elastic bands in the plurality of first elastic bands 201 ranges from 0.2 mm to 12 mm.

The plurality of second elastic bands 202 form a second group of elastic bands, which are arranged between the second base material 102 and the third base material 103, and the spacing between two adjacent elastic bands in the plurality of second elastic bands 202 ranges from 0.2 mm to 12 mm.

The plurality of third elastic bands 203 form a third group of elastic bands, which are arranged between the third base material 103 and the fourth base material 104, and the spacing between two adjacent elastic bands in the plurality of third elastic bands 203 ranges from 0.2 mm to 12 mm.

The projections of the plurality of first elastic bands 201, the plurality of second elastic bands 202, and the plurality of third elastic bands 203 are staggered in the vertical direction, such that the combined spacing D0 between adjacent elastic bands ranges from 0.1 mm to 6 mm.

The difference between Example 13 and Example 5 is that the number of the third elastic bands 203 in Example 13 is less than that in Example 5, which results in a plurality of combined spacings in Example 13, namely, combined spacings D01 and D02. This example can also change the final combined spacing, that is, in the same elastic layer, the combined spacings may be more than one.

### Example 14

As shown in Figure 6, the elastic layer for a wearable hygiene product in Example 14 comprises: a first base material 101, a second base material 102, a third base material 103, a fourth base material 104, a fifth base material 105, a plurality of first elastic bands 201, a plurality of second elastic bands 202, a plurality of third elastic bands 203, and a plurality of fourth elastic bands 204.

The first base material 101, the second base material 102, the third base material 103, the fourth base material 104, and the fifth base material 105 are stacked in sequence.

The plurality of first elastic bands 201 form a first group of elastic bands, which are arranged between the first base material 101 and the second base material 102, and the spacing between two adjacent elastic bands in the plurality of first elastic bands 201 ranges from 0.2 mm to 12 mm.

The plurality of second elastic bands 202 form a second group of elastic bands, which are arranged between the second base material 102 and the third base material 103, and the spacing between two adjacent elastic bands in the plurality of second elastic bands 202 ranges from 0.2 mm to 12 mm.

The plurality of third elastic bands 203 form a third group of elastic bands, which are arranged between the third base material 103 and the fourth base material 104, and the spacing between two adjacent elastic bands in the plurality of third elastic bands 203 ranges from 0.2 mm to 12 mm.

The plurality of fourth elastic bands 204 form a fourth group of elastic bands, which are arranged between the fourth base material 104 and the fifth base material 105, and the spacing between two adjacent elastic bands in the plurality of fourth elastic bands 204 ranges from 0.2 mm to 12 mm.

The projections of the plurality of first elastic bands 201, the plurality of second elastic bands 202, the plurality of third elastic bands 203, and the plurality of fourth elastic bands 204 are staggered in the vertical direction, such that the combined spacing D0 between adjacent elastic bands ranges from 0.1 mm to 6 mm.

The materials of the base material, the peel strength between adjacent base material, the average linear density, tensile range, and number of elastic bands in Examples 2 to 12 and 14 are similar to those in Example 1, and will not be repeated here.

In exemplary embodiments, at least one layer of the (N-2) layers of base material in the N layers of base material is discontinuous base material.

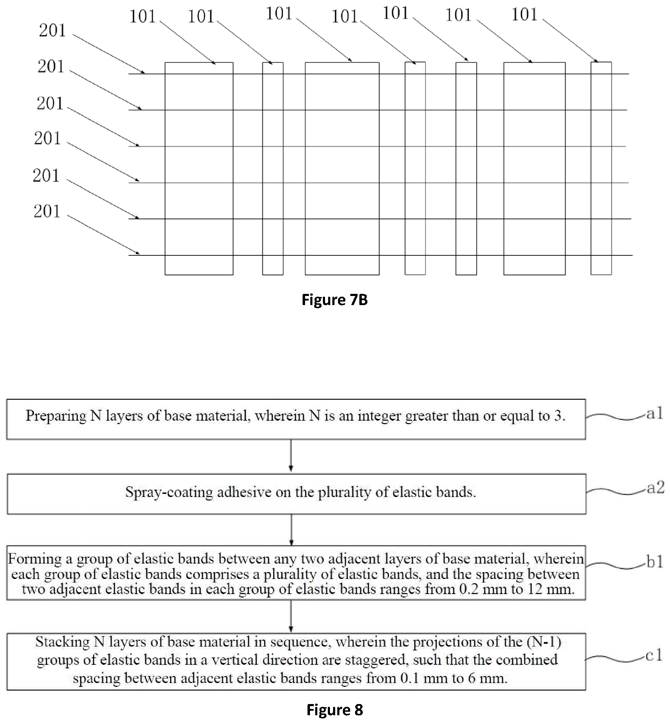

Figure 7A is a schematic diagram I of discontinuous base material, and Figure 7B is a schematic diagram II of discontinuous base material, both of which are viewed from top to bottom.

### Example 15

In Examples 1 to 14, each layer of base material is a continuous layer. In Example 15, as shown in Figures 7A and 7B, the first base material 101 is a discontinuous layer.

When N is 3, there may be one discontinuous layer of base material, which may be the first base material 101, or the second base material 102, or the third base material 103 in Figures 1A, 2A, 3A, and 4.

When N is 4, there may be one or two discontinuous layers of base material, which may be any one or any two layers of the first base material 101, the second base material 102, the third base material 103, and the fourth base material 104 in Figures 5A to 5I.

When N is 5, there may be one or two or three discontinuous layers of base material, which may be any one or any two or any three layers of the first base material 101, the second base material 102, the third base material 103, the fourth base material 104, and the fifth base material 105 in Figure 6.

Similarly, at least one layer of the (N-2) layers of base material in the N layers of base material is discontinuous base material.

The extension direction of the break lines of the discontinuous base material are perpendicular to the extension direction of the elastic bands, that is, the extension direction of a plurality of segments formed by the same layer of discontinuous base material is perpendicular to the extension direction of the elastic bands.

The number and size of the plurality of segments formed by the discontinuous base material may vary, for example, the number and size of segments formed by the first base material 101 after breaking in Figures 7A and 7B are different.

According to embodiments of the present disclosure, there is also provided a method for preparing an elastic layer for a wearable hygiene product. The relevant preparation methods will be introduced below with reference to the accompanying drawings.

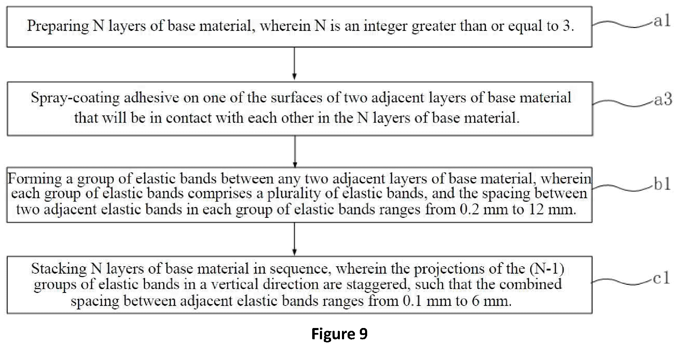

In exemplary embodiments, as shown in Figures 8 and 9, a method for preparing an elastic layer for a wearable hygiene product comprises:
Step a1) Preparing N layers of base material, wherein N is an integer greater than or equal to 3.
Step b1) Forming a group of elastic bands between any two adjacent layers of base material, wherein each group of elastic bands comprises a plurality of elastic bands, and the spacing between two adjacent elastic bands in each group of elastic bands ranges from 0.2 mm to 12 mm (e.g., 0.2 mm, 0.3 mm, 0.4 mm, 0.5 mm, 0.6 mm, 0.7 mm, 0.8 mm, 0.9 mm, 1 mm, 1.1 mm, 1.2 mm, 1.3 mm, 1.4 mm, 1.5 mm, 1.6 mm, 1.7 mm, 1.8 mm, 1.9 mm, 2 mm, 3 mm, 4 mm, 5 mm, 6 mm, 7 mm, 8 mm, 9 mm, 10 mm, 11 mm, 12 mm). Preferably, the spacing between two adjacent elastic bands in each group of elastic bands ranges from 2 mm to 12 mm.
Step c1) Stacking N layers of base material in sequence, wherein the projections of the (N-1) groups of elastic bands are staggered in the vertical direction, such that the combined spacing between adjacent elastic bands ranges from 0.1 mm to 6 mm (e.g., 0.1 mm, 0.2 mm, 0.3 mm, 0.4 mm, 0.5 mm, 0.6 mm, 0.7 mm, 0.8 mm, 0.9 mm, 1 mm, 1.5 mm, 2 mm, 2.5 mm, 3 mm, 3.5 mm, 4 mm, 4.5 mm, 5 mm, 5.5 mm, 6 mm). Preferably, the combined spacing ranges from 1 mm to 6 mm.

In exemplary embodiments, as shown in Figure 8, prior to forming a group of elastic bands between any two adjacent layers of base material (i.e., step b1), the method further comprises:
Step a2) Spray-coating adhesive on the plurality of elastic bands.

The elastic bands can be bonded to its two adjacent layers of base material through the adhesive, and each layer of base material is bonded to the adjacent base material through the adhesive spray-coated on the elastic bands.

In exemplary embodiments, as shown in Figure 9, prior to forming a group of elastic bands between any two adjacent layers of base material (i.e., step b1), the method further comprises:
Step a3) Spray-coating adhesive on one of the surfaces of two adjacent layers of base material that will be in contact with each other in the N layers of base material.

The elastic bands can be bonded to its two adjacent layers of base material through the adhesive, and two adjacent layers of base material can be bonded through the adhesive.

Taking three layers of base material and two groups of elastic bands as an example, there are four preparation methods.

The first method comprises:
Preparing three layers of base material which comprise a first base material 101, a second base material 102, and a third base material 103.

Laying a plurality of first elastic bands 201 on the upper surface of the first base material 101, wherein the spacing D1 between two adjacent elastic bands in the plurality of first elastic bands 201 ranges from 0.2 mm to 12 mm.

Laying a plurality of second elastic bands 202 on the lower surface of the third base material 103, wherein the spacing D2 between two adjacent elastic bands in the plurality of second elastic bands 202 ranges from 0.2 mm to 12 mm.

Stacking the second base material 102 on the side of the first base material where the first elastic bands 201 are laid (i.e., the upper surface of the first base material 101).

Laying the side of the third base material 103 where the second elastic bands 202 are laid on the second base material 102, wherein the projections of the plurality of first elastic bands 201 and the plurality of second elastic bands 202 in a vertical direction are staggered, and the combined spacing D0 between adjacent first elastic band 201 and second elastic band 202 ranges from 0.1 mm to 6 mm.

Thus, the elastic layer for a wearable hygiene product as shown in Figure 1A is prepared.

The second method comprises:
Preparing three layers of base material which comprise a first base material 101, a second base material 102, and a third base material 103.

Laying a plurality of first elastic bands 201 on the lower surface of the second base material 102, and laying a plurality of second elastic bands 202 on the upper surface of the second base material 102, wherein the spacing D1 between two adjacent elastic bands in the plurality of first elastic bands 201 ranges from 0.2 mm to 12 mm, the spacing D2 between two adjacent elastic bands in the plurality of second elastic bands 202 ranges from 0.2 mm to 12 mm, the projections of the plurality of first elastic bands 201 and the plurality of second elastic bands 202 in a vertical direction are staggered, and the combined spacing D0 between adjacent first elastic band 201 and second elastic band 202 ranges from 0.1 mm to 6 mm.

Stacking the second base material 102 on the first base material 101.

Stacking the third base material 103 on the second base material 102.

Thus, the elastic layer for a wearable hygiene product as shown in Figure 2A is prepared.

The third method comprises:
Preparing three layers of base material which comprise a first base material 101, a second base material 102, and a third base material 103.

Laying a plurality of first elastic bands 201 on the upper surface of the first base material 101, wherein the spacing D1 between two adjacent elastic bands in the plurality of first elastic bands 201 ranges from 0.2 mm to 12 mm.

Stacking the second base material 102 on the first base material 101.

Laying a plurality of second elastic bands 202 on the upper surface of the second base material 102, wherein the spacing D2 between two adjacent elastic bands in the plurality of second elastic bands 202 ranges from 0.2 mm to 12 mm, the projections of the plurality of first elastic bands 201 and the plurality of second elastic bands 202 in a vertical direction are staggered, and the combined spacing D0 between adjacent first elastic band and second elastic band ranges from 0.1 mm to 6 mm.

Stacking the third base material 103 on the second base material 102.

Thus, the elastic layer for a wearable hygiene product as shown in Figure 3A is prepared.

The fourth method comprises:
Preparing three layers of base material which comprise a first base material 101, a second base material 102, and a third base material 103.

Laying a plurality of first elastic bands 201 on the lower surface of the second base material 102, wherein the spacing D1 between two adjacent elastic bands in the plurality of first elastic bands 201 ranges from 0.2 mm to 12 mm.

Laying a plurality of second elastic bands 202 on the lower surface of the third base material 103, wherein the spacing D2 between two adjacent elastic bands in the plurality of second elastic bands 202 ranges from 0.2 mm to 12 mm.

Stacking the second base material 102 on the first base material 101, and stacking the third base material 103 on the second base material 102, wherein the projections of the plurality of first elastic bands 201 and the plurality of second elastic bands 202 in a vertical direction are staggered, and the combined spacing D0 between adjacent first elastic band 201 and second elastic band 202 ranges from 0.1 mm to 6 mm.

Thus, the elastic layer for a wearable hygiene product as shown in Figure 4 is prepared.

Taking four layers of base material and three groups of elastic bands as an example, there are eight preparation methods.

The first method comprises:
Preparing four layers of base material which comprise a first base material 101, a second base material 102, a third base material 103, and a fourth base material 104.

Laying a plurality of first elastic bands 201 on the upper surface of the first base material 101, wherein the spacing D1 between two adjacent elastic bands in the plurality of first elastic bands 201 ranges from 0.2 mm to 12 mm.

Stacking the second base material 102 on the first base material 101.

Laying a plurality of second elastic bands 202 on the upper surface of the second base material 102, wherein the spacing D2 between two adjacent elastic bands in the plurality of second elastic bands 202 ranges from 0.2 mm to 12 mm.

Stacking the third base material 103 on the second base material 102.

Laying a plurality of third elastic bands 203 on the upper surface of the third base material 103, wherein the spacing D3 between two adjacent elastic bands in the plurality of third elastic bands 203 ranges from 0.2 mm to 12 mm, the projections of the plurality of first elastic bands 201, the plurality of second elastic bands 202, and the plurality of third elastic bands 203 in a vertical direction are staggered, and the combined spacing D0 between adjacent first elastic band 201, second elastic band 202, and third elastic band 203 ranges from 0.1 mm to 6 mm.

Stacking the fourth base material 104 on the third base material 103.

Thus, the elastic layer for a wearable hygiene product as shown in Figure 5A is prepared.

The second method comprises:
Preparing four layers of base material which comprise a first base material 101, a second base material 102, a third base material 103, and a fourth base material 104.

Laying a plurality of first elastic bands 201 on the upper surface of the first base material 101, wherein the spacing D1 between two adjacent elastic bands in the plurality of first elastic bands 201 ranges from 0.2 mm to 12 mm.

Stacking the second base material 102 on the first base material 101.

Laying a plurality of second elastic bands 202 on the upper surface of the second base material 102, wherein the spacing D2 between two adjacent elastic bands in the plurality of second elastic bands 202 ranges from 0.2 mm to 12 mm.

Stacking the third base material 103 on the second base material 102.

Laying a plurality of third elastic bands 203 on the lower surface of the fourth base material 104, wherein the spacing D3 between two adjacent elastic bands in the plurality of third elastic bands 203 ranges from 0.2 mm to 12 mm.

Stacking the fourth base material 104 on the third base material 103, wherein the projections of the plurality of first elastic bands 201, the plurality of second elastic bands 202, and the plurality of third elastic bands 203 in a vertical direction are staggered, and the combined spacing D0 between adjacent first elastic band 201, second elastic band 202, and third elastic band 203 ranges from 0.1 mm to 6 mm.

Thus, the elastic layer for a wearable hygiene product as shown in Figure 5B is prepared.

The third method comprises:
Preparing four layers of base material which comprise a first base material 101, a second base material 102, a third base material 103, and a fourth base material 104.

Laying a plurality of first elastic bands 201 on the upper surface of the first base material 101, wherein the spacing D1 between two adjacent elastic bands in the plurality of first elastic bands 201 ranges from 0.2 mm to 12 mm.

Laying a plurality of second elastic bands 202 on the lower surface of the third base material 103, and laying a plurality of third elastic bands 203 on the upper surface of the third base material 103, wherein the spacing D2 between two adjacent elastic bands in the plurality of second elastic bands 202 ranges from 0.2 mm to 12 mm, and the spacing D3 between two adjacent elastic bands in the plurality of third elastic bands 203 ranges from 0.2 mm to 12 mm.

Stacking the second base material 102 on the first base material 101.

Stacking the third base material 103 on the second base material 102.

Stacking the fourth base material 104 on the third base material 103, wherein the projections of the plurality of first elastic bands 201, the plurality of second elastic bands 202, and the plurality of third elastic bands 203 in a vertical direction are staggered, and the combined spacing D0 between adjacent first elastic band 201, second elastic band 202, and third elastic band 203 ranges from 0.1 mm to 6 mm.

Thus, the elastic layer for a wearable hygiene product as shown in Figure 5C is prepared.

The fourth method comprises:
Preparing four layers of base material which comprise a first base material 101, a second base material 102, a third base material 103, and a fourth base material 104.

Laying a plurality of first elastic bands 201 on the upper surface of the first base material 101, wherein the spacing D1 between two adjacent elastic bands in the plurality of first elastic bands 201 ranges from 0.2 mm to 12 mm.

Laying a plurality of second elastic bands 202 on the lower surface of the third base material 103, wherein the spacing D2 between two adjacent elastic bands in the plurality of second elastic bands 202 ranges from 0.2 mm to 12 mm.

Laying a plurality of third elastic bands 203 on the lower surface of the fourth base material 104, wherein the spacing D3 between two adjacent elastic bands in the plurality of third elastic bands 203 ranges from 0.2 mm to 12 mm.

Stacking the second base material 102 on the first base material 101.

Stacking the third base material 103 on the second base material 102.

Stacking the fourth base material 104 on the third base material 103, wherein the projections of the plurality of first elastic bands 201, the plurality of second elastic bands 202, and the plurality of third elastic bands 203 in a vertical direction are staggered, and the combined spacing D0 between adjacent first elastic band 201, second elastic band 202, and third elastic band 203 ranges from 0.1 mm to 6 mm.

Thus, the elastic layer for a wearable hygiene product as shown in Figure 5D is prepared.

The fifth method comprises:
Preparing four layers of base material which comprise a first base material 101, a second base material 102, a third base material 103, and a fourth base material 104.

Laying a plurality of first elastic bands 201 on the lower surface of the second base material 102, and laying a plurality of second elastic bands 202 on the upper surface of the second base material 102, wherein the spacing D1 between two adjacent elastic bands in the plurality of first elastic bands 201 ranges from 0.2 mm to 12 mm, and the spacing D2 between two adjacent elastic bands in the plurality of second elastic bands 202 ranges from 0.2 mm to 12 mm.

Laying a plurality of third elastic bands 203 on the upper surface of the third base material 103, wherein the spacing D3 between two adjacent elastic bands in the plurality of third elastic bands 203 ranges from 0.2 mm to 12 mm.

Stacking the second base material 102 on the first base material 101.

Stacking the third base material 103 on the second base material 102.

Stacking the fourth base material 104 on the third base material 103, wherein the projections of the plurality of first elastic bands 201, the plurality of second elastic bands 202, and the plurality of third elastic bands 203 in a vertical direction are staggered, and the combined spacing D0 between adjacent first elastic band 201, second elastic band 202, and third elastic band 203 ranges from 0.1 mm to 6 mm.

Thus, the elastic layer for a wearable hygiene product as shown in Figure 5E is prepared.

The sixth method comprises:
Preparing four layers of base material which comprise a first base material 101, a second base material 102, a third base material 103, and a fourth base material 104.

Laying a plurality of first elastic bands 201 on the lower surface of the second base material 102, and laying a plurality of second elastic bands 202 on the upper surface of the second base material 102, wherein the spacing D1 between two adjacent elastic bands in the plurality of first elastic bands 201 ranges from 0.2 mm to 12 mm, and the spacing D2 between two adjacent elastic bands in the plurality of second elastic bands 202 ranges from 0.2 mm to 12 mm.

Laying a plurality of third elastic bands 203 on the lower surface of the fourth base material 104, wherein the spacing D3 between two adjacent elastic bands in the plurality of third elastic bands 203 ranges from 0.2 mm to 12 mm.

Stacking the second base material 102 on the first base material 101.

Stacking the third base material 103 on the second base material 102.

Stacking the fourth base material 104 on the third base material 103, wherein the projections of the plurality of first elastic bands 201, the plurality of second elastic bands 202, and the plurality of third elastic bands 203 in a vertical direction are staggered, and the combined spacing D0 between adjacent first elastic band 201, second elastic band 202, and third elastic band 203 ranges from 0.1 mm to 6 mm.

Thus, the elastic layer for a wearable hygiene product as shown in Figure 5F is prepared.

The seventh method comprises:
Preparing four layers of base material which comprise a first base material 101, a second base material 102, a third base material 103, and a fourth base material 104.

Laying a plurality of first elastic bands 201 on the lower surface of the second base material 102, wherein the spacing D1 between two adjacent elastic bands in the plurality of first elastic bands 201 ranges from 0.2 mm to 12 mm.

Laying a plurality of second elastic bands 202 on the lower surface of the third base material 103, and laying a plurality of third elastic bands 203 on the upper surface of the third base material 103, wherein the spacing D2 between two adjacent elastic bands in the plurality of second elastic bands 202 ranges from 0.2 mm to 12 mm, and the spacing D3 between two adjacent elastic bands in the plurality of third elastic bands 203 ranges from 0.2 mm to 12 mm.

Stacking the second base material 102 on the first base material 101.

Stacking the third base material 103 on the second base material 102.

Stacking the fourth base material 104 on the third base material 103, wherein the projections of the plurality of first elastic bands 201, the plurality of second elastic bands 202, and the plurality of third elastic bands 203 in a vertical direction are staggered, and the combined spacing D0 between adjacent first elastic band 201, second elastic band 202, and third elastic band 203 ranges from 0.1 mm to 6 mm.

Thus, the elastic layer for a wearable hygiene product as shown in Figure 5G is prepared.

The eighth method comprises:
Preparing four layers of base material which comprise a first base material 101, a second base material 102, a third base material 103, and a fourth base material 104.

Laying a plurality of first elastic bands 201 on the lower surface of the second base material 102, wherein the spacing D1 between two adjacent elastic bands in the plurality of first elastic bands 201 ranges from 0.2 mm to 12 mm.

Laying a plurality of second elastic bands 202 on the lower surface of the third base material 103, wherein the spacing D2 between two adjacent elastic bands in the plurality of second elastic bands 202 ranges from 0.2 mm to 12 mm.

Laying a plurality of third elastic bands 203 on the lower surface of the fourth base material 104, wherein the spacing D3 between two adjacent elastic bands in the plurality of third elastic bands 203 ranges from 0.2 mm to 12 mm.

Stacking the second base material 102 on the first base material 101.

Stacking the third base material 103 on the second base material 102.

Stacking the fourth base material 104 on the third base material 103, wherein the projections of the plurality of first elastic bands 201, the plurality of second elastic bands 202, and the plurality of third elastic bands 203 in a vertical direction are staggered, and the combined spacing D0 between adjacent first elastic band 201, second elastic band 202, and third elastic band 203 ranges from 0.1 mm to 6 mm.

Thus, the elastic layer for a wearable hygiene product as shown in Figure 5H is prepared.

Similarly, there are a plurality of preparation methods for the elastic layer for a wearable hygiene product consisting of five layers of base material and four groups of elastic bands. Herein, only one method will be introduced, which is as follows:
Preparing five layers of base material which comprise a first base material 101, a second base material 102, a third base material 103, a fourth base material 104, and a fifth base material 105.

Laying a plurality of first elastic bands 201 on the upper surface of the first base material 101, wherein the spacing D1 between two adjacent elastic bands in the plurality of first elastic bands 201 ranges from 0.2 mm to 12 mm.

Stacking the second base material 102 on the first base material 101.

Laying a plurality of second elastic bands 202 on the upper surface of the second base material 102, wherein the spacing D2 between two adjacent elastic bands in the plurality of second elastic bands 202 ranges from 0.2 mm to 12 mm.

Stacking the third base material 103 on the second base material 102.

Laying a plurality of third elastic bands 203 on the upper surface of the third base material 103, wherein the spacing D3 between two adjacent elastic bands in the plurality of third elastic bands 203 ranges from 0.2 mm to 12 mm.

Stacking the fourth base material 104 on the third base material 103.

Laying a plurality of fourth elastic bands 204 on the upper surface of the fourth base material 104, wherein the spacing D4 between two adjacent elastic bands in the plurality of fourth elastic bands 204 ranges from 0.2 mm to 12 mm, the projections of the plurality of first elastic bands 201, the plurality of second elastic bands 202, the plurality of third elastic bands 203, and the plurality of fourth elastic bands 204 in a vertical direction are staggered, and the combined spacing D0 between adjacent first elastic band 201, second elastic band 202, third elastic band 203, and fourth elastic band 204 ranges from 0.1 mm to 6 mm.

Stacking the fifth base material 105 on the fourth base material 104.

Thus, the elastic layer for a wearable hygiene product as shown in Figure 6 is prepared.

Other preparation methods for the elastic layer for a wearable hygiene product consisting of five layers of base material and four groups of elastic bands can refer to the preparation methods for the elastic layer for a wearable hygiene product consisting of four layers of base material and three groups of elastic bands, which will not be described in detail here.

By analogy, embodiments containing more layers of base material may be prepared.

The embodiments of the present disclosure also provide a wearable hygiene product, comprising the above-described elastic layers for a wearable hygiene product.

For convenience of interpretation and precise limitation of the appended claims., the terms "up", "down", "in", "out", "on", "below", "upside", "downside", "upward", "downward", "front", "back", "backward", "inside", "outside", "inward", "outward", "internal", "external", "internal", "external", "forwards", "backwards" are used to describe the features of the exemplary embodiments with reference to the positions of these features shown in the accompanying drawings.

The foregoing descriptions of the exemplary embodiments of the present disclosure are for the purpose of illustration and exemplification. The preceding description is not intended to be exhaustive, nor is it intended to limit the disclosure to the precise form disclosed. Obviously, many changes and variations are possible based on the above teachings. The purpose of selecting and describing exemplary embodiments is to explain the specific principles and practical applications of the present disclosure, so that others skilled in the art in the art can implement and utilize various exemplary embodiments of the present disclosure, as well as their different alternatives and modifications. The scope of the present disclosure is intended to be limited by the appended claims, and their equivalents.

## Claims

1. An elastic layer for a wearable hygiene product, **characterized in** comprising:
N layers of base material which are stacked in sequence, wherein N is an integer greater than or equal to 3; and
(N-1) groups of elastic bands, wherein each group of elastic bands among the (N-1) groups of elastic bands comprises a plurality of elastic bands;
wherein one group of elastic bands is provided between two adjacent layers of base material, and a spacing between two adjacent elastic bands in each group of elastic bands ranges from 0.2 mm to 12 mm, and projections of two adjacent groups of elastic bands in a vertical direction are staggered, such that a combined spacing between adjacent elastic bands ranges from 0.1 mm to 6 mm.

2. The elastic layer for a wearable hygiene product according to claim 1, **characterized in** comprising:
a first base material, a second base material, and a third base material which are stacked in sequence;
a plurality of first elastic bands forming a first group of elastic bands, which are arranged between the first base material and the second base material, wherein a spacing between two adjacent elastic bands in the plurality of first elastic bands ranges from 0.2 mm to 12 mm; and
a plurality of second elastic bands forming a second group of elastic bands, which are arranged between the second base material and the third base material, wherein a spacing between two adjacent elastic bands in the plurality of second elastic bands ranges from 0.2 mm to 12 mm;
wherein projections of the plurality of first elastic bands and the plurality of second elastic bands in a vertical direction are staggered, such that a combined spacing between adjacent first and second elastic bands ranges from 0.1 mm to 6 mm.

3. The elastic layer for a wearable hygiene product according to claim 1, **characterized in** comprising:
a first base material, a second base material, a third base material, and a fourth base material which are stacked in sequence;
a plurality of first elastic bands forming a first group of elastic bands, which are arranged between the first base material and the second base material, wherein a spacing between two adjacent elastic bands in the plurality of first elastic bands ranges from 0.2 mm to 12 mm;
a plurality of second elastic bands forming a second group of elastic bands, which are arranged between the second base material and the third base material, wherein a spacing between two adjacent elastic bands in the plurality of second elastic bands ranges from 0.2 mm to 12 mm; and
a plurality of third elastic bands forming a third group of elastic bands, which are arranged between the third base material and the fourth base material, wherein a spacing between two adjacent elastic bands in the plurality of third elastic bands ranges from 0.2 mm to 12 mm;
wherein projections of the plurality of first elastic bands, the plurality of second elastic bands, and the plurality of third elastic bands in a vertical direction are staggered, such that a combined spacing between adjacent elastic bands ranges from 0.1 mm to 6 mm.

4. The elastic layer for a wearable hygiene product according to claim 1, **characterized in** comprising:
a first base material, a second base material, a third base material, a fourth base material, and a fifth base material which are stacked in sequence;
a plurality of first elastic bands forming a first group of elastic bands, which are arranged between the first base material and the second base material, wherein a spacing between two adjacent elastic bands in the plurality of first elastic bands ranges from 0.2 mm to 12 mm;
a plurality of second elastic bands forming a second group of elastic bands, which are arranged between the second base material and the third base material, wherein a spacing between two adjacent elastic bands in the plurality of second elastic bands ranges from 0.2 mm to 12 mm;
a plurality of third elastic bands forming a third group of elastic bands, which are arranged between the third base material and the fourth base material, wherein a spacing between two adjacent elastic bands in the plurality of third elastic bands ranges from 0.2 mm to 12 mm; and
a plurality of fourth elastic bands forming a fourth group of elastic bands, which are arranged between the fourth base material and the fifth base material, wherein a spacing between two adjacent elastic bands in the plurality of fourth elastic bands ranges from 0.2 mm to 12 mm;
wherein projections of the plurality of first elastic bands, the plurality of second elastic bands, the plurality of third elastic bands, and the plurality of fourth elastic bands in a vertical direction are staggered, such that a combined spacing between adjacent elastic bands ranges from 0.1 mm to 6 mm.

5. The elastic layer for a wearable hygiene product according to claim 2, **characterized in that** the plurality of first elastic bands are distributed at equal intervals, and the plurality of second elastic bands are distributed at equal intervals.

6. The elastic layer for a wearable hygiene product according to claim 3, **characterized in that** the plurality of first elastic bands are distributed at equal intervals, the plurality of second elastic bands are distributed at equal intervals, and the plurality of third elastic bands are distributed at equal intervals.

7. The elastic layer for a wearable hygiene product according to claim 4, **characterized in that** the plurality of first elastic bands are distributed at equal intervals, the plurality of second elastic bands are distributed at equal intervals, the plurality of third elastic bands are distributed at equal intervals, and the plurality of fourth elastic bands are distributed at equal intervals.

8. The elastic layer for a wearable hygiene product according to claim 1, **characterized in that** the elastic band has an average linear density of 10 dtex to 440 dtex.

9. The elastic layer for a wearable hygiene product according to claim 1, **characterized in that** at least one layer of (N-2) layers of base material in the N layers of base material is a discontinuous base material.

10. A method for preparing an elastic layer for a wearable hygiene product, **characterized in** comprising:
preparing N layers of base material, wherein N is an integer greater than or equal to 3;
forming a group of elastic bands between any two adjacent layers of base material, each group of elastic bands comprising a plurality of elastic bands, wherein a spacing between two adjacent elastic bands in each group of elastic bands ranges from 0.2 mm to 12 mm; and
stacking the N layers of base material in sequence, wherein projections of (N-1) groups of elastic bands in a vertical direction are staggered, such that a combined spacing between adjacent elastic bands ranges from 0.1 mm to 6 mm.

11. The method for preparing an elastic layer for a wearable hygiene product according to claim 10, **characterized in that**, prior to forming a group of elastic bands between any two adjacent layers of base material, the method further comprises:
spray-coating adhesive on the plurality of elastic bands;
wherein the elastic bands are able to be bonded to its two adjacent layers of base material through adhesive, and each layer of base material is bonded to an adjacent layer of base material through the adhesive spray-coated on the elastic bands.

12. The method for preparing an elastic layer for a wearable hygiene product according to claim 10, **characterized in that**, prior to forming a group of elastic bands between any two adjacent layers of base material, the method further comprises:
spray-coating adhesive on one of surfaces of two adjacent layers of base material that will contact with each other;
wherein the elastic bands are able to be bonded to its two adjacent layers of base material through the adhesive, and the two adjacent layers of base material are bonded through the adhesive.

13. The method for preparing an elastic layer for a wearable hygiene product according to claim 10, **characterized in** comprising:
preparing three layers of base material which comprise a first base material, a second base material, and a third base material;
laying a plurality of first elastic bands on an upper surface of the first base material, wherein a spacing between two adjacent elastic bands in the plurality of first elastic bands ranges from 0.2 mm to 12 mm;
laying a plurality of second elastic bands on a lower surface of the third base material, wherein a spacing between two adjacent elastic bands in the plurality of second elastic bands ranges from 0.2 mm to 12 mm;
stacking the second base material on a side of the first base material where the first elastic bands are laid; and
stacking a side of the third base material where the second elastic bands are laid above the second base material, such that projections of the plurality of first elastic bands and the plurality of second elastic bands in a vertical direction are staggered, and a combined spacing between adjacent first and second elastic bands ranges from 0.1 mm to 6 mm.

14. The method for preparing an elastic layer for a wearable hygiene product according to claim 10, **characterized in** comprising:
preparing three layers of base material which comprise a first base material, a second base material, and a third base material;
laying a plurality of first elastic bands on a lower surface of the second base material, and laying a plurality of second elastic bands on an upper surface of the second base material, wherein a spacing between two adjacent elastic bands in the plurality of first elastic bands ranges from 0.2 mm to 12 mm, and a spacing between two adjacent elastic bands in the plurality of second elastic bands ranges from 0.2 mm to 12 mm, and projections of the plurality of first elastic bands and the plurality of second elastic bands in a vertical direction are staggered, and a combined spacing between adjacent first and second elastic bands ranges from 0.1 mm to 6 mm;
stacking the second base material on the first base material; and
stacking the third base material on the second base material.

15. The method for preparing an elastic layer for a wearable hygiene product according to claim 10, **characterized in** comprising:
preparing three layers of base material which comprise a first base material, a second base material, and a third base material;
laying a plurality of first elastic bands on an upper surface of the first base material, wherein a spacing between two adjacent elastic bands in the plurality of first elastic bands ranges from 0.2 mm to 12 mm;
stacking the second base material on the first base material;
laying a plurality of second elastic bands on an upper surface of the second base material, wherein a spacing between two adjacent elastic bands in the plurality of second elastic bands ranges from 0.2 mm to 12 mm, such that projections of the plurality of first elastic bands and the plurality of second elastic bands in a vertical direction are staggered, and a combined spacing between adjacent first and second elastic bands ranges from 0.1 mm to 6 mm; and
stacking the third base material on the second base material.

16. The method for preparing an elastic layer for a wearable hygiene product according to claim 10, **characterized in** comprising:
preparing four layers of base material which comprise a first base material, a second base material, a third base material, and a fourth base material;
laying a plurality of first elastic bands on an upper surface of the first base material, wherein a spacing between two adjacent elastic bands in the plurality of first elastic bands ranges from 0.2 mm to 12 mm;
laying a plurality of second elastic bands on a lower surface of the third base material, and laying a plurality of third elastic bands on an upper surface of the third base material, wherein a spacing between two adjacent elastic bands in the plurality of second elastic bands ranges from 0.2 mm to 12 mm, and a spacing between two adjacent elastic bands in the plurality of third elastic bands ranges from 0.2 mm to 12 mm;
stacking the second base material on the first base material;
stacking a side of the third base material where the second elastic bands are laid on the second base material; and
stacking the fourth base material on the third base material, such that projections of the plurality of first elastic bands, the plurality of second elastic bands, and the plurality of third elastic bands in a vertical direction are staggered, and a combined spacing between adjacent first, second, and third elastic bands ranges from 0.1 mm to 6 mm.

17. The method for preparing an elastic layer for a wearable hygiene product according to claim 10, **characterized in** comprising:
preparing four layers of base material which comprise a first base material, a second base material, a third base material, and a fourth base material;
laying a plurality of first elastic bands on a lower surface of the second base material, laying a plurality of second elastic bands on an upper surface of the second base material, laying a plurality of third elastic bands on a lower surface of the third base material, and laying a plurality of fourth elastic bands on an upper surface of the fourth base material, wherein a spacing between two adjacent elastic bands in the plurality of first elastic bands ranges from 0.2 mm to 12 mm, a spacing between two adjacent elastic bands in the plurality of second elastic bands ranges from 0.2 mm to 12 mm, and a spacing between two adjacent elastic bands in the plurality of third elastic bands ranges from 0.2 mm to 12 mm, wherein projections of the plurality of first elastic bands, the plurality of second elastic bands, and the plurality of third elastic bands in a vertical direction are staggered, and a combined spacing between adjacent first, second, and third elastic bands ranges from 0.1 mm to 6 mm;
stacking the second base material on the first base material;
stacking the third base material on the second base material; and
stacking the fourth base material on the third base material.

18. The method for preparing an elastic layer for a wearable hygiene product according to claim 10, **characterized in** comprising:
preparing four layers of base material which comprise a first base material, a second base material, a third base material, and a fourth base material;
laying a plurality of first elastic bands on an upper surface of the first base material, wherein a spacing between two adjacent elastic bands in the plurality of first elastic bands ranges from 0.2 mm to 12 mm;
stacking the second base material on the first base material;
laying a plurality of second elastic bands on an upper surface of the second base material, wherein a spacing between two adjacent elastic bands in the plurality of second elastic bands ranges from 0.2 mm to 12 mm;
stacking the third base material on the second base material;
laying a plurality of third elastic bands on an upper surface of the third base material, wherein a spacing between two adjacent elastic bands in the plurality of third elastic bands ranges from 0.2 mm to 12 mm, wherein projections of the plurality of first elastic bands, the plurality of second elastic bands, and the plurality of third elastic bands in a vertical direction are staggered, and a combined spacing between adjacent first, second and third elastic bands ranges from 0.1 mm to 6 mm; and
stacking the fourth base material on the third base material.

19. The method for preparing an elastic layer for a wearable hygiene product according to claim 10, **characterized in** comprising:
preparing five layers of base material which comprise a first base material, a second base material, a third base material, a fourth base material, and a fifth base material;
laying a plurality of first elastic bands on an upper surface of the first base material, wherein a spacing between two adjacent elastic bands in the plurality of first elastic bands ranges from 0.2 mm to 12 mm;
stacking the second base material on the first base material;
laying a plurality of second elastic bands on an upper surface of the second base material, wherein a spacing between two adjacent elastic bands in the plurality of second elastic bands ranges from 0.2 mm to 12 mm;
stacking the third base material on the second base material;
laying a plurality of third elastic bands on an upper surface of the third base material, wherein a spacing between two adjacent elastic bands in the plurality of third elastic bands ranges from 0.2 mm to 12 mm;
stacking the fourth base material on the third base material;
laying a plurality of fourth elastic bands on an upper surface of the fourth base material, wherein a spacing between two adjacent elastic bands in the plurality of fourth elastic bands ranges from 0.2 mm to 12 mm, wherein projections of the plurality of first elastic bands, the plurality of second elastic bands, the plurality of third elastic bands, and the plurality of fourth elastic bands in a vertical direction are staggered, and a combined spacing between adjacent first, second, third, and fourth elastic bands ranges from 0.1 mm to 6 mm; and
stacking the fifth base material on the fourth base material.
